# EUROPÄISCHE PATENTANMELDUNG

(11) **EP 3 127 942 A1**
(43) Veröffentlichungstag der Anmeldung: **08.02.2017**
(21) Anmeldenummer: 15179556.4
(22) Anmeldetag: 03.08.2015
(51) Int. Cl.: C08J 3/09, C09D 167/03, C09D 171/12, C09D 179/08, C09D 181/06, C07C 39/26, C07C 19/04, C07C 19/045

(54) **POLYMERLÖSUNG, VERFAHREN ZUR HERSTELLUNG UND VERWENDUNG EINER SOLCHEN POLYMERLÖSUNG**

(71) Anmelder: Novamem AG, 8006 Zürich (CH)
(72) Erfinder: Schumacher, Christoph Martin, 9000 St. Gallen (CH); Loepfe, Michael, 8006 Zürich (CH)
(74) Vertreter: Hepp Wenger Ryffel AG

(57) **Zusammenfassung**

Die Erfindung betrifft eine Polymerlösung. Die Polymerlösung umfasst ein zumindest binäres Lösungsmittelsystem und mindestens ein Polymer. Das Lösungsmittelsystem umfasst ein organisches Lösungsmittel und ein Additiv. Das mindestens eine Polymer ist im organischen Lösungsmittel lösbar. Das Additiv erniedrigt die Schmelztemperatur der Polymerlösung und ist oder umfasst eine halogenierte organische Verbindung, die das Polymer oder die Polymere nicht löst. Die Erfindung betrifft auch ein Verfahren zur Herstellung der Polymerlösung, die Verwendung einer solchen Polymerlösung, ein Lösungsmittelsystem sowie ein Additiv.

## Beschreibung

Die Erfindung betrifft eine Polymerlösung, ein Verfahren zur Herstellung der Polymerlösung sowie ihre Verwendung gemäss den Oberbegriffen der unabhängigen Ansprüche.

In nahezu allen Industriebereichen werden immer höhere Anforderungen an die verwendeten Materialen gestellt. Besonders Werkstoffe mit einer hohen mechanischen Robustheit und sehr guter Resistent gegenüber gängigen Chemikalien finden breite Anwendung. Eine Klasse von Werkstoffen, welche diese Anforderungen erfüllen, stellen sogenannte Hochleistungspolymere, wie beispielsweise PEEK (Polyetheretherketon), dar.

PEEK ist ein teilweise kristallines und hochschmelzendes Polymer. Es zeichnet sich insbesondere durch seine hohe Chemikalienbeständigkeit, Temperaturbeständigkeit sowie mechanischen Eigenschaften aus, was eine Vielzahl von Einsatzmöglichkeiten nach sich zieht. Typischerweise wird PEEK mittels Extrusionsverfahren oder Pressverfahren verarbeitet. Es sind jedoch nur wenige Lösungsmittel bekannt, welche das Polymer lösen. Solche Lösungsmittel haben zudem den Nachteil, dass sie unter Standardbedingungen fest sind. Darüber hinaus können mit diesen Lösungsmitteln keine stabilen Lösungen hergestellt werden. Um stabile Lösungen herzustellen, sind weitere Hilfsmassnahmen, wie beispielsweise Wärmeschränke, notwendig. Das wiederum erschwert jedoch den Einsatz solcher Lösungen bei industriellen Verfahren, wie beispielsweise der Rollbeschichtung, da kein Kontakt mit Bauteilen, welche eine geringe Temperatur als die Erstarrungstemperatur der Lösung aufweisen, erfolgen darf. Dadurch wird der Einsatzbereich von PEEK und anderen Hochleistungspolymeren eingeschränkt.

Aus US 7 439 291 sind Polymerlösungen bekannt, welche ein teilweise kristallines und lösungsmittelinertes Polymer in einem eutektischen Lösungsmittelsystem umfassen. Das eutektische Gemisch umfasst mindestens zwei Lösungsmittel. Jedes Lösungsmittel für sich hat einen Schmelzpunkt oberhalb von 20°C, das eutektische Gemisch weist einen Schmelzpunkt unterhalb von 20°C auf. Bei dem ersten Lösungsmittel handelt es sich um ein halogeniertes Phenolderivat, welche das lösungsmittelinerte Polymer bei erhöhter Temperatur löst. Das zweite Lösungsmittel ist, abgesehen von 4-Chloro-2-methyl-phenol, ein nichthalogeniertes Phenolderivat, insbesondere *p*-Cresol. Durch diese Zusammensetzung wird eine giessbare Polymerlösung erhalten, aus der lösungsmittelresistente Membranen bei Raumtemperatur gegossen werden können. Auch PEEK kann mittels dieses Lösungsmittelsystems vorübergehend in Lösung gebracht werden.

Diese Polymerlösungen haben jedoch den Nachteil, dass sie nicht dauerhaft stabil sind und vor der Weiterverarbeitung frisch zubereitet werden müssen. Temperaturen unterhalb von 25°C werden nicht toleriert. Da diese Polymerlösungen für jede Anwendung neu hergestellt werden müssen, ist mit einem hohen Arbeitsaufwand und hohen Kosten für industrielle Anwendungen zu rechnen. Aufgrund der Unhandlichkeit und den Kosten ist die Verwendung solcher Polymerlösungen bei der Herstellung von Bauteilen aller Art und Beschichtungen, beispielsweise in der Automobil-Industrie, nicht wirtschaftlich.

Es ist daher Aufgabe der Erfindung, die Nachteile des Standes der Technik zu überwinden.

Insbesondere ist es eine Aufgabe der vorliegenden Erfindung, eine Polymerlösung zur Verfügung zu stellen, welche dauerhaft stabil ist und mit geringem Arbeitsaufwand und kostengünstig hergestellt werden kann. Es ist eine weitere Aufgabe der Erfindung ein Verfahren zur Herstellung einer solchen Polymerlösung sowie eine Verwendung bereitzustellen.

Diese Aufgaben werden durch die in den unabhängigen Patentansprüchen definierten Polymerlösung und Verfahren sowie Verwendung gelöst.

Die Erfindung betrifft eine Polymerlösung, welche ein zumindest binäres Lösungsmittelsystem und mindestens ein Polymer umfasst. Das zumindest binäre Lösungsmittelsystem umfasst ein organisches Lösungsmittel und ein Additiv. Das mindestens eine Polymer ist im organischen Lösungsmittel lösbar. Das Additiv erniedrigt die Schmelztemperatur der Polymerlösung und ist oder umfasst eine halogenierte organische Verbindung, die das mindestens eine Polymer nicht löst.

Als "nichtlösend" wird verstanden, wenn sich weniger als 1 g Polymer in 1 kg Lösungsmittel bei 25° C unter Standarddruck (1 bar) lösen. Typischerweise weist das organische Lösungsmittel einen Schmelztemperatur auf, welche gleich oder höher 20°C ist. Das mindestens eine Polymer löst sich unter Erwärmen in dem organischen Lösungsmittel. Sobald das organische Lösungsmittel und das mindestens eine Polymer eine homogene Lösung bilden, wird das Additiv der Lösung zugefügt. Die Polymerlösung kann bei Zugabe des Additivs eine Temperatur um 25°C oder höher aufweisen. Die Angaben der Schmelztemperaturen beziehen sich im Rahmen der Erfindung stets auf den Standarddruck (1 bar).

Das Additiv kann das mindestens eine Polymer für sich nicht lösen, verhindert aber eine sichtbare Präzipitation des Polymers aus der Polymerlösung, insbesondere, wenn die Polymerlösung bei Temperaturen um 25°C oder tiefer aufbewahrt wird. Es stabilisiert die Polymerlösung. Des Weiteren wird die Schmelztemperatur der Polymerlösung durch die Zugabe des Additivs gesenkt.

Eine solche Polymerlösung zeichnet sich dadurch aus, dass das mindestens eine Polymer in einem breiten Temperaturbereich in der Polymerlösung verbleibt und unter verschiedenen Bedingungen verarbeitet werden kann. Die Polymerlösung kann über mehrere Wochen und Monate gelagert werden, bevor diese zur Weiterverarbeitung verwendet wird. Dadurch ist es möglich auch grössere Mengen der Polymerlösung herzustellen, was den Einsatz in industriellen Verfahren einerseits erleichtert und andererseits den Arbeitsaufwand und die Kosten deutlich verringert. Zudem werden durch das Bereitstellen der Polymerlösung neue Verfahrenstechniken, wie etwa das Sprühverfahren, zugänglich.

Vorzugsweise ist das mindestens eine Polymer in einem Temperaturbereich von -20°C bis 25°C dauerhaft im zumindest binären Lösungsmittelsystem gelöst. Der Temperaturbereich von -20°C bis 25°C bezieht sich auf den Standarddruck (1 bar). Unter "dauerhaft gelöst" ist eine Zeitspanne von mindestens 2 Wochen gemeint. Bei Polymerlösungen mit einem Polymeranteil bis 4.5 Gew.% kann das mindestens eine Polymer bei derartigen Lösungen über mehrere Jahre in Lösung verbleiben.

Eine derart dauerhaft stabile Lösung hat den Vorteil, dass sie in grösseren Mengen hergestellt werden kann, da diese nicht gleich nach der Herstellung weiterverarbeitet werden muss. Dadurch können industrielle Herstellungsprozesse hinsichtlich Kosten und Arbeitsaufwand optimiert werden.

In der Polymerlösung liegt das mindestens eine Polymer vorzugsweise in einer Endkonzentration von 0.1 bis10 Gewichtsprozent (Gew.%), besonders bevorzugt 2 bis 7 Gew.% und ganz besonders bevorzugt 3 bis 6 Gew.% vor. In Abhängigkeit vom verwendeten Polymer oder Polymeren haben sich Polymerlösungen mit 3, 4, und 4.5 Gew.% als besonders vorteilhaft ergeben. Es ist aber auch denkbar Polymerlösung mit einem Gehalt bis 12 Gew.% herzustellen, welche zumindest für einen kurzen Zeitraum bei Standardbedingungen verarbeitet werden können. Unter Standardbedingungen versteht man eine Temperatur von 25°C und ein Druck von 1 bar.

Polymerlösungen solcher Konzentrationen zeichnen sich dadurch aus, dass sie sich besonders gut verarbeiten lassen. Aufgrund des breiten tolerierten Konzentrationsbereiches der Polymerlösung kann diese ihren verschiedenen Verwendungen angepasst werden.

Die Polymerlösung kann dabei ein Mengenverhältnis (in Gewichtsprozent) an organischem Lösungsmittel und Additiv zwischen 33:67 und 95:5 aufweisen. Vorzugsweise liegen das organische Lösungsmittel und das Additiv in einem Verhältnis von 67:33 vor.

Vorzugsweise liegt das organische Lösungsmittel in einer grösseren Menge vor als das Additiv. Es ist aber auch denkbar, dass das Additiv in einer grösseren Menge vorliegt.

Durch die Einstellung des Verhältnisses an organischem Lösungsmittel und Additiv können unterschiedliche Mengen an Polymer oder Polymeren dauerhaft in Lösung gehalten werden. Da das Additiv darüber hinaus auch die Schmelztemperatur der Polymerlösung absenkt, kann eine lagerstabile Lösung über einen breiten Temperaturbereich bereitgestellt werden, was wiederum vielseitige Verwendungsmöglichkeiten bietet.

Das Additiv kann eine niedrigere Schmelztemperatur als das organische Lösungsmittel aufweisen, vorzugsweise eine Schmelztemperatur unterhalb von 25°C. Besonders tiefe Schmelztemperaturen weisen dabei Dichlormethan und Chloroform mit -97°C respektive - 63°C auf.

In Kombination mit einem höher schmelzenden organischen Lösungsmittel kann somit eine Polymerlösung erzielt werden, deren Schmelztemperatur deutlich unterhalb von 25°C liegt. Schmelztemperaturen unterhalb von 0°C sind insbesondere mit Chloroform und Dichlormethan möglich.

Das mindestens eine Polymer kann ein zumindest teilweise kristallines Polymer sein. Bevorzugt ist das mindestens eine Polymer ein aliphatisches und/oder aromatisches Polyketon und ganz besonders bevorzugt Polyetheretherketon (PEEK). Unter "teilweise kristallin" wird verstanden, dass amorphe und kristalline Bereiche im festen Zustand des Polymers neben einander vorliegen.

PEEK ist ein thermoplastischer Kunststoff mit einer sehr hohen Schmelztemperatur von 335°C. PEEK gehört zu den Hochleistungskunststoffen. Hochleistungskunststoffe zeichnen sich durch eine hohe Temperaturbeständigkeit, Chemikalienbeständigkeit und besondere mechanische Eigenschaften aus. PEEK ist gegen fast alle organischen und anorganischen beständig. Bis 280°C ist der Kunststoff auch gegen Hydrolyse beständig. Aufgrund seiner Robustheit findet PEEK eine Vielzahl von Anwendungen. So wird PEEK beispielsweise bei der Herstellung von Pumpen, HPLC-Säulen, Ventile, Bauteile für Automotoren und Kabelisolationen verwendet. Es ist zudem eines der wenigen Kunststoffe, die sich für Anwendungen unter Ultrahochvakuum eignen.

Das mindestens eine Polymer ist jedoch nicht auf Polyetheretherketon beschränkt. Denkbar wäre beispielsweise auch die Verwendung von Polyimid-Verbindungen wie beispielsweise Polyimide auf der Basis von Polyetherimid (PEI) oder Polysulfone (PSU).

Bei dem mindestens einem Polymer kann es sich um ein flüssigkristallines Polymer (LCP) handeln. Technisch bedeutsam sind beispielsweise aromatische Polyester (Vectra®/Zenite® LCP von Celanese, Xydar® LCP von Solvay, Sumikasuper LCP von Sumitomo Chemical). Diese LCPs weisen eine sehr hohe thermische Stabilität auf und sind chemisch sehr beständig. Als Polymerfolien und Polymerfilme sind sie für Gase und Wasserdampf nur sehr schlecht durchdringbar und weisen eine hohe Barrierewirkung auf. Ein Quellverhalten ist in den meisten Lösungsmitteln kaum vorhanden. Als Fasern besitzen LCPs eine sehr hohe mechanische Stabilität. Vectra® ist ein Co-Polymer aus 4-Hydroxybenzoesäure und 6-Hydroxy-Napthaliensäure.

Das Lösen von LCPs und die daraus resultierenden Polymerlösungen ermöglichen einen breiteren Einsatz von LCPs und die Entwicklung neuer Materialien auf Basis von LCPs.

Das organische Lösungsmittel kann mindestens ein substituiertes Phenol sein oder umfassen, vorzugsweise 4-Chlorphenol, Pentafluorophenol, 2,3,5,6-Tetrafluorophenol, 3,5-Bis(trifluoromethyl)phenol, 4-Chlor-2-methylphenol oder 4-Chlor-3-methyl-phenol oder Kombinationen davon. Vorzugsweise handelt es sich bei den substituierten Phenolen um ein- oder mehrfach halogenierte Phenole.

Das Additiv kann eine ein- oder mehrfach halogenierte organische Verbindung sein oder umfassen, vorzugsweise 1,2,4-Trichlorobenzen, Dichlormethan, Chloroform, Chlorbenzol, Hexafluoroisopropanol, 2-Chlorphenol, Dichlorethan, Trichlorethan oder Tetrachlorethan oder Kombinationen davon. Das Additiv weist dabei eine Schmelztemperatur auf, die unterhalb von 25°C liegt. Das Additiv kann das Polymer allein nicht lösen.

Als besonders gute Zusammensetzungen für die Polymerlösung haben sich insbesondere Mischungen herausgestellt, welche circa 4 Gew.% PEEK und folgende Kombinationen aus organischem Lösungsmittel und Additiv im Verhältnis 2:1 umfassen:
- 4-Chlorphenol und 1,2,4-Trichlorbenzol
- Pentafluorphenol und 1,2,4-Trichlorbezol
- 3,5-Bis(trifluormethyl)phenol und 1,2,4-Trichlorbenzol
- 4-Chloro-2-methylphenol und 1,2,4-Trichlorobenzene
- 4-Chloro-3-methylphenol und 1,2,4-Trichlorobenzene

Für die Herstellung von LCP-Lösungen waren Kombinationen aus:
- Pentafluorpehnol und 1,2,4-Trichlorbenzol,
- 3,5-Bis(trifluormethyl)phenol und 1,2,4-Trichlorbenzol,
- 2,3,5,6-Tetrafluorphenol und 1,2,4-Trichlorbenzol
mit einem Verhältnis von 2:1 (organisches Lösungsmittel/Additiv) und 3 Gew.% LCP besonders bevorzugt.

Umfasst das zumindest binäre Lösungsmittelsystem 4-Chlorphenol als organisches Lösungsmittel und 1,2,4-Trichlorbenzol als Additiv so kann es vorteilhaft sein, das organische Lösungsmittel und das Additiv in einem Verhältnis von 33:67 (1:2) einzusetzen, um eine dauerhaft stabile Polymerlösung zu erhalten.

Polymerlösungen mit Chloroform und/oder Dichlormethan als Additiv waren auch über einen längeren Zeitraum (mindestens 2 Wochen) bei -20°C stabil. Es wurde keine Präzipitation des Polymers oder der Polymere beobachtet.

Die Kombination aus organischem Lösungsmittel und Additiv hat den Vorteil, dass, insbesondere durch die Zugabe des Additivs, die Polymerlösung stabilisiert wird. Eine Päzipitation des Polymers oder der Polymere wird über einen breiten Temperaturbereich verhindert, was wiederum ein breites Einsatzspektrum ermöglicht.

Die Wahl des Additivs ist dabei essentiell. In Hinblick auf US 7 439 291 haben Vergleichsstudien ergeben, dass die Kombination aus organischem Lösungsmittel und Additiv, welche je für sich das Polymer lösen, keine dauerhaft stabilen Polymerlösungen liefern. So konnten beispielsweise Lösungen aus 4-Chlor-2-Methylphenol und 4-Chlorphenol nicht bei Raumtemperatur gelagert werden ohne die Präzipitation des Poylmers zu beoachten. Ein ähnliches Ergebnis wurde beobachtet, wenn *p*-Cresol als Additiv verwendet wurde.

Ein weiterer Aspekt der Erfindung bezieht sich auf ein Verfahren zum Herstellen einer Polymerlösung, insbesondere einer Polymerlösung wie vorliegend beschrieben. Beim Herstellen der Polymerlösung werden mindestens ein Polymer, mindestens ein organisches Lösungsmittel und mindestens ein Additiv zusammen gemischt.

Das organische Lösungsmittel wird dabei zunächst zusammen mit dem mindestens einen Polymer solange erwärmt bis eine homogene Lösung aus Polymer und organischem Lösungsmittel resultiert. Nach Abkühlen der Lösung auf eine Temperatur unterhalb der Siedetemperatur des gewünschten Additivs, wird dieses der Lösung beigemischt.

Als organische Lösungsmittel eignen sich insbesondere substituiertes Phenole, vorzugsweise 4-Chlorphenol, Pentafluorophenol, 2,3,5,6-Tetrafluorophenol, 3,5-Bis(trifluoromethyl)phenol, 4-Chlor-2-methylphenol oder 4-Chlor-3-methyl-phenol oder Kombinationen davon. Vorzugsweise handelt es sich bei den substituierten Phenolen um ein- oder mehrfach halogenierte Phenole.

Das Additiv kann eine ein- oder mehrfach halogenierte organische Verbindung sein oder umfassen, vorzugsweise 1,2,4-Trichlorobenzen, Dichlormethan, Chloroform, Chlorbenzol, Hexafluoroisopropanol, 2-Chlorphenol, Dichlorethan, Trichlorethan oder Tetrachlorethan oder Kombinationen davon.

Die Herstellung einer solchen Polymerlösung hat den Vorteil, dass sie kostengünstig und mit wenig Arbeitsaufwand verbunden ist. Die erhaltenen Polymerlösungen zeichnen sich durch ihre Lagerstabilität aus.

Vorzugsweise liegt das organische Lösungsmittel in einer Menge (Gewichtsprozent) zwischen 33 und 95 Gewichtsprozent vor, vorzugsweise mit 67 Gewichtsprozenten.

Das mindestens eine Polymer kann ein zumindest teilweise kristallines Polymer, bevorzugt ein aliphatisches und/oder aromatisches Polyketon, und ganz besonders bevorzugt Polyetheretherketon (PEEK), sein. Aber auch LCPs, wie beispielsweise Vectra®, sind denkbar.

Als besonders gute Zusammensetzungen für die Polymerlösung haben sich insbesondere Mischungen herausgestellt, welche circa 4 Gew.% PEEK und folgende Kombinationen aus organischem Lösungsmittel und Additiv im Verhältnis 2:1 umfassen:
- 4-Chlorphenol und 1,2,4-Trichlorbenzol,
- Pentafluorphenol und 1,2,4-Trichlorbezol,
- 3,5-Bis(trifluormethyl)phenol und 1,2,4-Trichlorbenzol,
- 4-Chloro-2-methylphenol und 1,2,4-Trichlorobenzene,
- 4-Chloro-3-methylphenol und 1,2,4-Trichlorobenzene.

Für die Herstellung von LCP-Lösungen waren Kombinationen aus
- Pentafluorpehnol und 1,2,4-Trichlorbenzol,
- 3,5-Bis(trifluormethyl)phenol und 1,2,4-Trichlorbenzol,
- 2,3,5,6-Tetrafluorphenol und 1,2,4-Trichlorbenzol,
mit einem Verhältnis von 2:1 (organisches Lösungsmittel/Additiv) und 3 Gew.% LCP besonders bevorzugt.

Umfasst das zumindest binäre Lösungsmittelsystem 4-Chlorphenol als organisches Lösungsmittel und 1,2,4-Trichlorbenzol als Additiv so kann es vorteilhaft sein, das organische Lösungsmittel und das Additiv in einem Verhältnis von 33:67 (1:2) einzusetzen, um eine dauerhaft stabile Polymerlösung zu erhalten.

Polymerlösungen mit Chloroform und/oder Dichlormethan als Additiv waren auch über einen längeren Zeitraum (mindestens 2 Wochen) bei -20°C stabil. Es wurde keine Präzipitation des Polymers oder der Polymere beobachtet.

Ein weiterer Aspekt der Erfindung betrifft ein Lösungsmittelsystem. Das Lösungsmittelsystem umfassend ein organisches Lösungsmittel und ein Additiv. Das Additiv ist oder umfasst eine organische, halogenierte Verbindung und liegt in einer Menge zwischen 5 und 67 Gewichtsprozent, vorzugsweise 33 Gewichtsprozent, vor. Das organische Lösungsmittel kann in einer Menge zwischen 33 und 67 Gewichtsprozent vorliegen. Es ist denkbar, dass das Additiv einen grösseren Mengenanteil im Lösungsmittelsystem ausmacht, als das organische Lösungsmittel. Das organische Lösungsmittel ist oder umfasst vorzugsweise ein substituiertes Phenol.

Ein solches Lösungsmittelsystem hat den Vorteil, dass eine Vielzahl von schwer löslichen oder unlöslichen Verbindungen, insbesondere Polymeren, einerseits in Lösungen gebracht werden können und andererseits in dieser gehalten werden können. Dadurch ergibt sich die Möglichkeit Polymere für neue Verarbeitungsverfahren zugänglich zu machen und eine Vielzahl von Anwendungen bereitzustellen.

Ein weiterer Aspekt der Erfindung betrifft die Verwendung einer Polymerlösung, insbesondere einer Polymerlösung wie vorliegend beschrieben, zur Herstellung einer Polymermembran, Folien, Verpackungen und Beschichtungen für Bauteile aller Art sowie Isolierungen.

LCPs zeichnen sich besonders durch seine hohen Barriereeigenschaften, insbesondere gegenüber Wasserdampf und Gasen, aus. Dies kann insbesondere bei der Herstellung von Verpackungen für den Pharmaziebereich von Bedeutung sein, da in diesem Bereich häufig sterile und langlebige, gegen äussere Einflüsse resistente Verpackungen benötigt werden und durch die Verwendung von LCP-haltigen Verpackungen bereitgestellt werden können. Aber auch der Einsatz solcher Polymerlösung bei der Herstellung von medizintechnisches Equipment ist möglich. PEEK ist ein weiteres Polymer, das in den Polymerlösungen verwendet werden kann.

Aber auch die Verwendung solcher Polymerlösung für lösungsmittelresistente nano- bis mikroporösen Membranen ist denkbar. Solche Membrane können in unterschiedlichen Dicken und mit unterschiedlichen Porengrössen hergestellt werden, wodurch ein breites Anwendungsspektrum ermöglicht wird, beispielsweise bei der Trennung von Flüssigkeiten und Gasen. Herstellungsverfahren solcher Membranen sind beispielsweise aus WO 2012/097967 bekannt.

Des Weiteren ist die Verwendung einer solchen Polymerlösung für die Herstellung verschiedenster Bauteile und Beschichtungen in der Automobil- und Flugzeugindustrie möglich.

Die Verwendung einer solchen Polymerlösung hat den Vorteil, dass die hohen Verarbeitungstemperaturen, die beispielsweise zum Schmelzen von PEEK notwendig sind, umgangen werden können und eine energieeffiziente Herstellung verschiedenster Produkte ermöglicht wird. Des Weiteren wird der Zugang zu neuen Verarbeitungsverfahren ermöglicht, beispielsweise dem Sprühverfahren oder der Rollbeschichtung. Solche Verfahren konnten bisher nicht auf PEEK aufgrund seiner hohen Schmelztemperatur und der Viskosität angewendet werden.

Ein weiterer Aspekt der Erfindung betrifft die Verwendung eines Additivs zur Herstellung einer dauerhaft stabilen Polymerlösung. Die Polymerlösung basiert auf einem organischen Lösungsmittel und mindestens einem Polymer. Das mindestens eine Polymer ist im organischen Lösungsmittel lösbar. Das Additiv erniedrigt die Schmelztemperatur der Polymerlösung und ist oder umfasst eine halogenierte organische Verbindung, die das mindestens eine Polymer nicht löst.

Die Erfindung wird im Folgenden anhand von Ausführungsbeispielen näher erläutert.

| | |
|---|---|
| Beispiel 1-6: | erfindungsgemässe Polymerlösungen mit PEEK, welche bei Standardbedingungen (25°C, 1 bar) dauerhaft stabil sind |
| Beispiel 7-8: | erfindungsgemässe Polymerlösungen mit PEEK, welche bis -20°C unter Standardbedingungen (1 bar) dauerhaft stabil sind. |
| Beispiel 9-10: | erfindungsgemässe Polymerlösung mit Vectra® LCP, welche bei Standardbedingungen (25°C, 1 bar) dauerhaft stabil sind. |
| Beispiele 11-12: | Vergleichsbeispiele |

### Beispiel 1 Poly(etheretherketone) PEEK, grade 90G, wurde von Victrex Manufacturing Ltd., Thornton-Cleveleys, Lancashire, UK, bezogen. Pentafluorphenol (99+ %) wurde von Fluorochem Ltd., Hadfield, Derbyshire, UK, bezogen.

Eine Mischung aus Pentafluorphenol und 6 Gew.% Polymer wurde auf 50 °C erhitzt, um das Lösungsmittel zu schmelzen. Anschliessend wurde die Lösung gerührt und auf 140 °C erhitzt, bis sich das Polymer komplett gelöst hatte. Danach wurde die klare Lösung auf 120 °C gekühlt und 1,2,4-Trichlorbenzol (≥ 99 %), bezogen von Sigma-Aldrich GmbH, Buchs, Schweiz, zugegeben, um eine Endkonzentration des Polymers von 4 Gew.% zu erhalten. Es resultierte eine bei Raumtemperatur stabile und giessbare Lösung.

### Beispiel 2 Poly(etheretherketone) PEEK, grade 600G, wurde von Victrex Manufacturing Ltd., Thornton-Cleveleys, Lancashire, UK, bezogen. Pentafluorphenol (99+ %) wurde von Fluorochem Ltd., Hadfield, Derbyshire, UK, bezogen.

Eine Mischung aus Pentafluorphenol und 4.5 Gew.% Polymer wurde auf 50 °C erhitzt, um das Lösungsmittel zu schmelzen. Anschliessend wurde die Lösung gerührt und auf 140 °C erhitzt, bis sich das Polymer komplett gelöst hatte. Danach wurde die klare Lösung auf 55 °C gekühlt und Chloroform (≥ 99.8 %), bezogen von Sigma-Aldrich GmbH, Buchs, Schweiz, zugegeben, um eine Endkonzentration des Polymers von 3 Gew.% zu erhalten. Es resultierte eine bei Raumtemperatur stabile und giessbare Lösung.

### Beispiel 3 Poly(etheretherketone) PEEK, grade 450G, wurde von from Victrex Manufacturing Ltd., Thornton-Cleveleys, Lancashire, UK, bezogen. 3,5-Bis(trifluormethyl)phenol (98 %) wurde von from Apollo Scientific Ltd., Bredbury, Stockport, Cheshire, UK, bezogen.

Eine Mischung aus 3,5-Bis(trifluormethyl)phenol und 6 Gew.% Polymer wurde gerührt und auf 150 °C erhitzt, bis sich das Polymer komplett gelöst hatte. Danach wurde die klare Lösung auf 70 °C gekühlt und 1,2-Dichlorethan (≥ 99 %), bezogen von Sigma-Aldrich GmbH, Buchs, Schweiz, zugegeben, um eine Endkonzentration des Polymers von 4 Gew.% zu erhalten. Es resultierte eine bei Raumtemperatur stabile und giessbare Lösung.

*Beispiel 4* Poly(etheretherketone) PEEK, grade 380G, wurde von Victrex Manufacturing Ltd., Thornton-Cleveleys, Lancashire, UK, bezogen. 3,5-Bis(trifluormethyl)phenol (98 %) wurde von Apollo Scientific Ltd., Bredbury, Stockport, Cheshire, UK, bezogen.

Eine Mischung aus 3,5-Bis(trifluormethyl)phenol und 6 Gew.% Polymer wurde gerührt und auf 150 °C erhitzt, bis sich das Polymer komplett gelöst hatte. Danach wurde die klare Lösung auf 120 °C gekühlt und 1,1,2,2-Tetrachlorethan (≥ 98 %), bezogen von Sigma-Aldrich GmbH, Buchs, Schweiz, zugegeben, um eine Endkonzentration des Polymers von 4 Gew.% zu erhalten. Es resultierte eine bei Raumtemperatur stabile und giessbare Lösung.

### Beispiel 5 Poly(etherketoneetherketoneketone) PEKEKK, grade STG45, wurde from Victrex Manufacturing Ltd., Thornton-Cleveleys, Lancashire, UK, bezogen. 4-Chlorphenol (> 98 %) wurde von TCI Europe N.V., Zwijndrecht, Belgien, bezogen.

Eine Mischung aus 4-Chlorphenol und 6 Gew.% Polymer wurde auf 50 °C erhitzt, um das Lösungsmittel zu schmelzen. Anschliessend wurde die Lösung gerührt und auf 210 °C erhitzt, bis sich das Polymer komplett gelöst hatte. Danach wurde die klare Lösung auf 55 °C gekühlt und Hexafluor-2-propanol (≥ 99.9 %), bezogen von Fluorochem Ltd., Hadfield, Derbyshire, United Kingdom, zugegeben, um eine Endkonzentration des Polymers von 4 Gew.% zu erhalten. Es resultierte eine bei Raumtemperatur stabile und giessbare Lösung.

### Beispiel 6 Poly(etheretherketone) PEEK, grade 150G, wurde von Victrex Manufacturing Ltd., Thornton-Cleveleys, Lancashire, UK, bezogen. 4-Chlorophenol (> 98 %) wurde von TCI Europe N.V., Zwijndrecht, Belgien, bezogen.

Eine Mischung aus 4-chlorphenol und 3 Gew.% Polymer wurde auf 50 °C erhitzt, um das Lösungsmittel zu schmelzen. Anschliessend wurde die Lösung gerührt und auf 210 °C erhitzt, bis sich das Polymer komplett gelöst hatte. Danach wurde die klare Lösung auf 120 °C gekühlt und Chlorbenzol (≥ 99.5 %), bezogen von Sigma-Aldrich GmbH, Buchs, Schweiz, zugegeben, um eine Endkonzentration des Polymers von 2 Gew.% zu erhalten. Es resultierte eine bei Raumtemperatur stabile und giessbare Lösung.

### Beispiel 7 Poly(etheretherketone) PEEK, grade 450G, wurde von Victrex Manufacturing Ltd., Thornton-Cleveleys, Lancashire, UK, bezogen. 4-Chlorophenol (> 98 %) wurde von TCI Europe N.V., Zwijndrecht, Belgien, bezogen.

Eine Mischung aus 4-Chlorphenol und 6 Gew.% Polymer wurde auf 50 °C erhitzt, um das Lösungsmittel zu schmelzen. Anschliessend wurde die Lösung gerührt und auf 210 °C erhitzt, bis sich das Polymer komplett gelöst hatte. Danach wurde die klare Lösung auf 40 °C gekühlt und Chloroform (≥ 99.8 %), bezogen von Sigma-Aldrich GmbH, Buchs, Schweiz, zugegeben, um eine Endkonzentration des Polymers von 4 Gew.% zu erhalten. Die klare Lösung wurde auf -20 °C gekühlt. Es resultierte eine stabile und giessbare Lösung. Nach 4 Wochen wurde keine Präzipitation des Polymers beobachtet.

### Beipsiel 8 Poly(etheretherketone) PEEK, grade 450G, wurde von Victrex Manufacturing Ltd., Thornton-Cleveleys, Lancashire, UK, bezogen. 4-Chlorophenol (> 98 %) wurde von TCI Europe N.V., Zwijndrecht, Belgien, bezogen.

Eine Mischung aus 4-Chlorphenol und 6 Gew.% Polymer wurde auf 50 °C erhitzt, um das Lösungsmittel zu schmelzen. Anschliessend wurde die Lösung gerührt und auf 210 °C erhitzt, bis sich das Polymer komplett gelöst hatte. Danach wurde die klare Lösung auf 35 °C gekühlt und Dichlormethan (≥ 99.8 %), bezogen von Sigma-Aldrich GmbH, Buchs, Schweiz, zugegeben, um eine Endkonzentration des Polymers von 4 Gew.% zu erhalten. Die klare Lösung wurde auf -20 °C gekühlt. Es resultierte eine stabile und giessbare Lösung. Nach 4 Wochen wurde keine Präzipitation des Polymers beobachtet.

### Beispiel 9 Flüssigkristallpolymer (LCP), Vectra grade A950, wurde von Celanese, Irving, Texas, USA, bezogen. Pentafluorphenol (99+ %) wurde von Fluorochem Ltd., Hadfield, Derbyshire, UK, bezogen.

Eine Mischung aus Pentafluorphenol und 3 Gew.% Polymer wurde auf 50 °C erhitzt, um das Lösungsmittel zu schmelzen. Anschliessend wurde die Lösung gerührt und auf 140 °C erhitzt, bis sich das Polymer komplett gelöst hatte. Danach wurde die klare Lösung auf 120°C gekühlt und 1,2,4-Trichlorbenzol (≥ 99 %), bezogen von Sigma-Aldrich GmbH, Buchs, Schweiz, zugegeben, um eine Endkonzentration des Polymers von 2 Gew.% zu erhalten. Es resultierte eine bei Raumtemperatur stabile und giessbare Lösung.

### Example 10 Flüssigkristallpolymer (LCP), Vectra grade A950, wurde von Celanese, Irving, Texas, USA, bezogen. 3,5-Bis(trifluormethyl)phenol (98 %) wurde von Apollo Scientific Ltd., Bredbury, Stockport, Cheshire, UK, bezogen.

Eine Mischung aus 3,5-Bis(trifluormethyl)phenol und 3 Gew.% Polymer wurde gerührt und auf 100 °C erhitzt, bis sich das Polymer komplett gelöst hatte. Danach wurde die klare Lösung auf 55 °C gekühlt und Chloroform (≥ 99.8 %), bezogen von Sigma-Aldrich GmbH, Buchs, Schweiz, zugegeben, um eine Endkonzentration des Polymers von 2 Gew.% zu erhalten. Es resultierte eine bei Raumtemperatur stabile und giessbare Lösung.

### Example 11 Poly(etheretherketone) PEEK, grade 450G, wurde von Victrex Manufacturing Ltd., Thornton-Cleveleys, Lancashire, UK, bezogen. 4-Chloro-2-Methylphenol (97 %) wurde von Sigma-Aldrich GmbH, Buchs, Schweiz, bezogen.

Eine Mischung aus 4-Chloro-2-Methylphenol und 6 Gew.% Polymer wurde auf 50 °C erhitzt, um das Lösungsmittel zu schmelzen. Anschliessend wurde die Lösung gerührt und auf 210 °C erhitzt, bis sich das Polymer komplett gelöst hatte. Danach wurde die klare Lösung auf 120°C gekühlt und *p*-Cresol (99 %), bezogen von Sigma-Aldrich GmbH, Buchs, Schweiz, zugegeben, um eine Endkonzentration des Polymers von 4 Gew.% zu erhalten. Die klare Lösung wurde auf Raumtemperatur (25°C) gekühlt. Während der Abkühlung präzipitierte das Polymer.

### Beispiel 12 Poly(etheretherketone) PEEK, grade 450G, wurde von Victrex Manufacturing Ltd., Thornton-Cleveleys, Lancashire, UK, bezogen. 4-Chloro-2-Methylphenol (> 90 %) wurde von TCI Europe N.V., Zwijndrecht, Belgien, bezogen.

Eine Mischung aus 4-Chloro-2-Methylphenol und 5.3 Gew.% Polymer wurde auf 50 °C erhitzt, um das Lösungsmittel zu schmelzen. Anschliessend wurde die Lösung gerührt und auf 210 °C erhitzt, bis sich das Polymer komplett gelöst hatte. Danach wurde die klare Lösung auf 120°C gekühlt und 4-Chlorphenol (98 %), bezogen von TCI Europe N.V., Zwijndrecht, Belgien, zugegeben, um eine Endkonzentration des Polymers von 4 Gew.% zu erhalten. Die klare Lösung wurde auf Raumtemperatur (25 °C) gekühlt. Das Lösungsmittel kristallisierte, was zur Präzipitation des Polymers nach erneutem Schmelzen auf 50 °C, führte.

## Patentansprüche

1. Polymerlösung, umfassend ein zumindest binäres Lösungsmittelsystem und mindestens ein Polymer, wobei das zumindest binäre Lösungsmittelsystem ein organisches Lösungsmittel und ein Additiv umfasst, das mindestens eine Polymer im organischen Lösungsmittel lösbar ist, und wobei das Additiv die Schmelztemperatur der Polymerlösung erniedrigt, **dadurch gekennzeichnet, dass** das Additiv eine halogenierte organische Verbindung ist oder umfasst, die das mindestens eine Polymer nicht löst.

2. Polymerlösung nach Anspruch 1, wobei das mindestens eine Polymer in einem Temperaturbereich von -20°C bis 25°C dauerhaft im zumindest binären Lösungsmittelsystem gelöst ist.

3. Polymerlösung nach Anspruch 1 oder 2, wobei das mindestens eine Polymer in einer Konzentration von 0.1 bis 10 Gewichtsprozent, vorzugsweise 2 bis 7 Gewichtsprozent und ganz besonders bevorzugt 3 bis 6 Gewichtsprozent vorliegt.

4. Polymerlösung nach einem der Ansprüche 1 bis 3, wobei die Menge (Gewichtsprozent) an organischem Lösungsmittel und Additiv ein Verhältnis zwischen 33:67 und 95:5 aufweist, vorzugsweise ein Verhältnis von 67:33.

5. Polymerlösung nach einem der Ansprüche 1 bis 4, wobei das Additiv einen niedrigere Schmelztemperatur als das organische Lösungsmittel aufweist, vorzugsweise eine Schmelztemperatur unterhalb von 25°C.

6. Polymerlösung nach einem der vorherigen Ansprüche, wobei das mindestens eine Polymer ein zumindest teilweise kristallines Polymer, bevorzugt ein aliphatisches und/oder aromatisches Polyketon, und ganz besonders bevorzugt Polyetheretherketon (PEEK), ist.

7. Polymerlösung nach einem der vorherigen Ansprüche, wobei das mindestens ein Polymer ein flüssigkristallines Polymer (LCP) ist.

8. Polymerlösung nach einem der vorherigen Ansprüche, wobei das organische Lösungsmittel mindestens ein substituiertes Phenol ist oder umfasst, vorzugsweise 4-Chlorphenol, Pentafluorophenol, 2,3,5,6-Tetrafluorphenol, 3,5-Bis(trifluoromethyl)phenol, 4-Chlor-2-methylphenol oder 4-Chlor-3-methyl-phenol oder Kombinationen davon.

9. Polymerlösung nach einem der vorherigen Ansprüche, wobei das Additiv eine ein- oder mehrfach halogenierte organische Verbindung ist oder umfasst, vorzugsweise 1,2,4-Trichlorobenzen, Dichlormethan, Chloroform, Chlorbenzol, Hexafluoroisopropanol, 2-Chlorphenol, Dichlorethan, Trichlorethan oder Tetrachlorethan oder Kombinationen davon.

10. Verfahren zum Herstellen einer Polymerlösung , insbesondere einer Polymerlösung nach einem der Ansprüche 1 bis 9, wobei mindestens ein Polymer, mindestens ein organisches Lösungsmittel und mindestens ein Additiv zusammen gemischt werden.

11. Verfahren nach Anspruch 10, wobei das organische Lösungsmittel in einer Menge (Gewichtsprozent) zwischen 33 und 95 Gewichtsprozent vorliegt, vorzugsweise mit 67 Gewichtsprozenten.

12. Verfahren nach einem der Ansprüche 10 oder 11, wobei das mindestens eine Polymer ein zumindest teilweise kristallines Polymer, bevorzugt ein aliphatisches und/oder aromatisches Polyketon, und ganz besonders bevorzugt Polyetheretherketon (PEEK), ist.

13. Lösungsmittelsystem umfassend ein organisches Lösungsmittel und ein Additiv, wobei das Additiv eine organische, halogenierte Verbindung ist oder umfasst und in einer Menge zwischen 5 und 67 Gewichtsprozent, vorzugsweise 33 Gewichtsprozent, vorliegt.

14. Verwendung einer Polymerlösung, insbesondere einer Polymerlösung nach einem der Ansprüche 1 bis 9, zur Herstellung einer Polymermembran, Folien, Verpackungen und Beschichtungen für Bauteile aller Art und medizinische Geräte.

15. Verwendung eines Additivs zur Herstellung einer dauerhaft stabilen Polymerlösung wobei die Polymerlösung auf einem organischen Lösungsmittel und mindestens einem Polymer basiert, wobei das mindestens eine Polymer im organischen Lösungsmittel lösbar ist, wobei das Additiv die Schmelztemperatur der Polymerlösung erniedrigt und eine halogenierte organische Verbindung ist oder umfasst, die das mindestens eine Polymer nicht löst.
